(19) Europäisches Patentamt
European Patent Office
Office européen des brevets

(11) Publication number : 0 061 799 B1

(12) # EUROPEAN PATENT SPECIFICATION

(45) Date of publication of patent specification : 07.11.84

(51) Int. Cl.[3] : C 01 B 33/28, B 01 J 29/30, C 07 C 5/22, C 07 C 5/32

(21) Application number : 82200304.2

(22) Date of filing : 09.03.82

(54) **Crystalline silicates.**

(30) Priority : 30.03.81 NL 8101543

(43) Date of publication of application : 06.10.82 Bulletin 82/40

(45) Publication of the grant of the patent : 07.11.84 Bulletin 84/45

(84) Designated contracting states : AT BE CH DE FR GB IT LI SE

(56) References cited :
DE-A- 1 667 727
GB-A- 1 484 495
US-A- 3 702 886
US-A- 3 770 845
US-E- 29 948

(73) Proprietor : SHELL INTERNATIONALE RESEARCH MAATSCHAPPIJ B.V.
Carel van Bylandtlaan 30
NL-2596 HR Den Haag (NL)

(72) Inventor : Lucien, Jacques Pierre
Badhuisweg 3
NL-1031 CM Amsterdam (NL)
Inventor : Post, Martin Franciscus Maria
Badhuisweg 3
NL-1031 CM Amsterdam (NL)

(74) Representative : Puister, Antonius Tonnis, Mr. et al
P.O. Box 302
NL-2501 CH The Hague (NL)

EP 0 061 799 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

**Description**

The invention relates to new crystalline silicates and also to a process for the preparation of such silicates. The invention further relates to the application of these silicates, in particular as catalysts in the isomerization of n-paraffins to form iso-paraffins and in the dehydrogenation of paraffins to form olefins.

Research on crystalline silicates has recently led to the preparation of new crystalline metal silicates having a special structure. The said crystalline metal silicates are characterized in that after one hour's calcination in air at 500 °C they have the following properties :

1) thermally stable up to a temperature of at least 600 °C,

2) an X-ray powder diffraction pattern in which the four lines listed in Table A are the strongest lines

Table A

| d (Å) | Relative intensity |
|---|---|
| 11.1 ± 0.2 | VS |
| 10.0 ± 0.2 | VS |
| 3.84 ± 0.07 | S |
| 3.72 ± 0.06 | S |

in which the letters used have the following meanings : VS = very strong ; S = strong,

3) in the formula which represents the composition of the silicate, expressed in moles of the oxides and which, in addition to oxides of hydrogen, alkali metal and/or alkaline earth metal and silicon, contains one or more oxides of a trivalent metal A, chosen from the group formed by aluminium, iron, gallium, rhodium, chromium and scandium, the $SiO_2/A_2O_3$ molar ratio is higher than 10.

In the present patent application a crystalline silicate having a thermal stability of at least t °C should be taken to be a silicate whose X-ray powder diffraction pattern does not change substantially upon heating to a temperature of t °C.

The above-mentioned crystalline metal silicates of a special structure are extremely valuable compositions. The can be used as adsorbing and extracting agents, as drying agents, as ion exchangers and as catalysts or catalyst carriers in carrying out a variety of catalytic processes. Examples of processes which can be successfully carried out using the above-mentioned crystalline metal silicates as catalysts, are : the preparation of aromatic hydrocarbons from aliphatic organic compounds, such as methanol, dewaxing of hydrocarbon oils, such as gas oil, cracking of heavy hydrocarbon oils and the preparation of p-xylene by methylation or disproportionation of toluene.

The Applicant has carried out an investigation into the application of crystalline metal silicates of the afore-mentioned type as catalysts in the isomerization of n-paraffins to form iso-paraffins and in the dehydrogenation of paraffins to form olefins. The catalytic isomerization of n-paraffins to form iso-paraffins plays a significant role in gasoline manufacture. Unbranched paraffins, such as n-pentane and n-hexane have a low octane number and, therefore, are not very suitable as gasoline components. By contacting them at elevated temperature which an isomerization catalyst, the n-paraffins are partly converted into iso-paraffins having the same number of carbon atoms, which have a higher octane number and are therefore more suitable as gasoline components.

The catalytic dehydrogenation of paraffins to form olefins likewise plays a significant part in gasoline manufacture. The olefins formed may be converted into alkylate gasoline by reaction with iso-paraffins, or, by contact with certain catalysts, they may be converted into an aromatic hydrocarbon mixture boiling in the gasoline range. The olefins formed may also be used as an alkylating agent for the preparation of alkyl aromatics. By contacting paraffins, such as propane, n-butane and isobutane, at elevated temperature with a dehydrogenation catalyst they are partly converted into the corresponding monoolefins.

The main criteria on which the judgment of the suitability of catalysts for the purposes in question is based, are the isomerizing selectivity for an isomerization catalyst and the dehydrogenating selectivity for a dehydrogenation catalyst. Besides, in both cases the cracking selectivity plays a very important role.

Taking a n-paraffin and a paraffin having a given number of carbon atoms as the respective feeds, the said criteria are defined as follows : the isomerizing and the dehydrogenating selectivity, respectively, are the weight ratios of the quantity of, respectively, iso-paraffins and olefins formed having the same number of carbon atoms as the respective n-paraffin and paraffin feeds, to the quantity of, respectively, n-paraffin feed and paraffin feed converted ; the cracking selectivity is the weight ratio of the quantity of hydrocarbons formed having a lower number of carbon atoms than the n-paraffin feed and the paraffin feed, respectively, to the quantity of, respectively, n-paraffin feed and paraffin feed converted. A catalyst is considered to be more suitable as an isomerization catalyst or as a dehydrogenation catalyst according as the isomerizing selectivity or, respectively, the dehydrogenating selectivity is higher and the cracking selectivity is lower.

The investigation carried out by the Applicant has shown that the afore-mentioned crystalline metal

silicates are not very suitable as catalysts, neither in the isomerization of n-paraffins to form iso-paraffins nor in the dehydrogenation of paraffins to form olefins. They have a very low isomerizing and dehydrogenating selectivity, while their cracking selectivity is very high.

Upon continued investigations into this subject by the Applicant, as a class of compounds has been found which compounds are closely related to the above-mentioned crystalline metal silicates and which compounds are very suitable for use as catalysts for isomerizing n-paraffins to form iso-paraffins and for dehydrogenating paraffins to form olefins. The compounds, which hereinafter will be referred to as crystalline cobalt silicates, have after one hour's calcination in air at 500 °C the following properties :

a) thermally stable up to a temperature of at least 600 °C,

b) an X-ray powder diffraction pattern in which the four lines listed in Table A are the strongest lines,

c) in the formula which represents the composition of the silicate, expressed in moles of the oxides, and which, in addition to oxides of hydrogen, alkali metal and/or alkaline earth metal, silicon and trivalent cobalt, optionally contains one or more oxides of a trivalent metal A chosen from the group formed by aluminium, iron, gallium, rhodium, chromium and scandium, the $SiO_2/(Co_2O_3 + A_2O_3)$ molar ratio (hereinafter designated m) is higher than 10 and the $A_2O_3/Co_2O_3$ molar ratio is lower than 1.

The crystalline cobalt silicates defined hereinabove, when used for isomerizing n-paraffins to form iso-paraffins, show a high isomerizing selectivity and when used for dehydrogenating paraffins to form olefins show a high dehydrogenating selectivity. They have a low cracking selectivity. The crystalline cobalt silicates are new substances.

The present patent application therefore relates to new crystalline metal silicates which have after one hour's calcination in air at 500 °C the properties mentioned in a)-c). The patent application further relates to their preparation and to their application, in particular their application as catalysts in the isomerization of n-paraffins to form iso-paraffins and their application as catalysts in the dehydrogenation of paraffins to form olefins.

Although in principle the crystalline cobalt silicates according to the invention may, in addition to trivalent cobalt, contain one or more other trivalent metals A, silicates which contain only cobalt as the trivalent metal are preferred for the applications mentioned above. As regards the presence of aluminium in the cobalt silicates according to the invention, the following should be noted. The silicon compounds eligible from the economical point of fiew for the preparation of crystalline silicates on a technical scale, generally contain a small quantity of aluminium as an impurity. Generally, at least part of this aluminium is found in the ready silicate. This means that if crystalline silicates according to the invention, containing cobalt either alone or in combination with iron, gallium, rhodium, chromium and/or scandium, are to be prepared starting from a mixture, an aluminium-contaminated silicon compound, then, as a rule, a crystalline cobalt silicate according to the invention containing a minor quantity of aluminium will be obtained.

In the overall formula of the silicates according to the invention m should have a value which lies above 10. For catalytic applications preference is given to silicates having an overall formula where m is smaller than 1,000 and in particular smaller than 400 and to silicates having an overall formula where m is larger than 20.

The crystalline cobalt silicates are defined, inter alia, by the X-ray powder diffraction pattern that the silicate shows after one hour's calcination in air at 500 °C. In this pattern the strongest lines should be the four lines listed in Table A. The complexe X-ray powder diffraction pattern of a typical example of a cobalt silicate according to the invention is given in Table B.

Table B

| d(Å) | Rel. int. | d(Å) | Rel. int. |
|---|---|---|---|
| 11.1 | 100 | 3.84 (D) | 57 |
| 10.0 (D) | 70 | 3.70 (D) | 31 |
| 8.93 | 1 | 3.63 | 16 |
| 7.99 | 1 | 3.47 | 1 |
| 7.42 | 2 | 3.43 | 5 |
| 6.68 | 7 | 3.34 | 2 |
| 6.35 | 11 | 3.30 | 5 |
| 5.97 | 17 | 3.25 | 1 |
| 5.70 | 7 | 3.05 | 8 |
| 5.56 | 10 | 2.98 | 11 |
| 5.35 | 2 | 2.96 | 3 |

(Continued)

| d(Å) | Rel. int. | d(Å) | Rel. int. |
|---|---|---|---|
| 4.98 (D) | 6 | 2.86 | 2 |
| 4.60 | 4 | 2.73 | 2 |
| 4.35 | 5 | 2.60 | 2 |
| 4.25 | 7 | 2.48 | 3 |
| 4.07 | 2 | 2.40 | 2 |
| 4.00 | 4 | | |

(D) = doublet

The crystalline cobalt silicates according to the invention can be prepared starting from an aqueous mixture containing the following compounds : one or more compounds of an alkali metal or alkaline earth metal (M), one or more compounds containing an organic cation (R) or from which such a cation is formed during the preparation of the silicate, one or more silicon compounds, one or more compounds containing cobalt in the trivalent form and, if desired, one or more compounds containing a trivalent metal A. The preparation is carried out by maintaining the mixture at an elevated temperature until the silicate has formed and subsequently separating the silicate crystals from the mother liquor. The aqueous mixture from which the silicates are prepared should contain the various compounds in the following ratios, expressed in moles of the oxides :

$M_{2/n}O : SiO_2 = 0.01\text{-}0.35$,
$R_{2/q}O : SiO_2 = 0.01\text{-}0.4$,
$SiO_2 : (Co_2O_3 + A_2O_3) > 10$,
$A_2O_3 : Co_2O_3 < 1$ and
$H_2O : SiO_2 = 5\text{-}65$

(n is the valency of M and q is the valency of R).

In addition to the new silicates the present patent application also relates to a process for the preparation of the new silicates starting from an aqueous mixture having the afore-mentioned composition, by maintaining this mixture at an elevated temperature until the silicate has formed and subsequently isolating the silicate from the mother liquor. The preparation of the silicates according to the invention may be carried out both at atmospheric and at elevated pressure. If reaction temperatures are used which lie above the boiling point of the mixture, the preparation is preferably carried out in an autoclave, at autogenous pressure. The silicates are preferably prepared by maintaining the mixture for at least four hours at a temperature between 90 and 300 °C and in particular at a temperature between 125 and 175 °C. After the silicates have formed, the crystals are separated from the mother liquor, for instance by filtration, decantation or centrifugation. Then the mass of crystals is washed with water and finally dried at a temperature between 100 and 200 °C.

As examples of suitable compounds that can be used in the preparation of the silicates according to the invention may be mentioned nitrates, carbonates, hydroxides and oxides of alkali metals and alkaline earth metals ; primary, secondary and tertiary alkylamines and quarternary alkylammonium compounds, such as bromides and hydroxides ; heterocyclic nitrogen compounds, such as pyridine and piperidine ; sodium silicate, silicic acid and amorphous silicas ; aluminium hydroxide and and sodium aluminate ; gallium oxide and gallium nitrate and compounds containing cobalt, iron, scandium rhodium and chromium in the trivalent form, such as the oxides, the hydroxides, normal salts, in particular the nitrates, and complex salts. In the preparation of the silicates according to the invention the starting mixture is preferably a mixture in which M is present in an alkali metal compound and R in an tetraalkyl ammonium compound and in particular a starting mixture in which M is present in a sodium compound R in an tetrapropyl ammonium compound.

Silicates prepared by heating the mixtures mentioned above contain alkali metal and/or alkaline earth metal ions and organic cations. By means of suitable exchange methods the alkali metal and alkaline earth metal ions can be replaced by other cations, such as hydrogen ions, ammonium ions or ions of the rare earth metals. Organic cations can very suitably be converted into hydrogen ions by calcination of the silicates.

If the silicates according to the invention contain alkali metal and the silicates are to be used as catalysts or catalyst carriers, the alkali metal content of these silicates should preferably be reduced beforehand to less than 1 %w and in particular to less than 0.05 %w. The reduction of the alkali metal

content of the silicates can very suitably be carried out by contacting them once or several times with an aqueous solution containing ammonium ions. From the $NH_4^+$ silicates thus obtained the $H^+$ silicates can be prepared by calcination.

Silicates according to the invention have catalytic activity and can therefore be used as catalysts such as they are. They may also be used as catalyst carriers for one more catalytically active metal components. In order to improve their catalytic performance promoters may be incorporated.

When the silicates according to the invention are to be used for catalytic purposes, they should, as a rule, be available in the form of particles having a diameter of 0.5-5 mm. The method of preparation described hereinbefore yields silicates in the form of a fine powder. The silicates can be shaped to form particles of a larger size, for instance by pressing. During shaping the silicates may be combined with a binder material, such as kaoline or bentonite. For the sake of brevity, catalysts that consist at least partly of a silicate according to the invention will hereinafter be designated « catalysts according to the invention ».

Although the catalysts according to the invention have a long life, they have to be regenerated from time to time. This can be done in a simple manner by burning off.

Catalysts according to the invention are of special interest in the isomerization of n-paraffins to form iso-paraffins and in the dehydrogenation of paraffins to form olefins.

As already observed hereinbefore, the catalytic isomerization of n-paraffins to form iso-paraffins plays an important role in gasoline manufacture. The n-paraffins which are subjected to the isomerization treatment preferably contain at least five and in particular five or six carbon atoms per molecule. The isomerization of the low-molecular-weight n-paraffins mentioned above using the catalyst according to the invention can be carried out at a temperature of 400-600 °C, a pressure of 1-10 bar and a space velocity of 0.1-10 $g \cdot g^{-1} \cdot h^{-1}$. The isomerization of low-molecular-weight n-paraffins is an attractive method of upgrading light gasoline fractions, such as tops obtained by straight distillation. Any iso-paraffins also present in such gasoline fractions can be removed before isomerization. Since the isomerization of n-paraffins is an equilibrium reaction, the product issuing from the isomerization reactor will still contain a quantity of unconverted n-paraffins. These may be separated from the isomerized product and recycled to the isomerization process. It is also possible to add the isomerized product to the original mixture of n-paraffins and iso-paraffins, to remove the iso-paraffins from this mixture and to isomerize the remaining mixture of n-paraffins according to the invention. Crystalline cobalt silicates according to the invention, when used as catalysts for the isomerization of n-paraffins having five or six carbon atoms in the molecule, have the property of converting part of the n-paraffin into a mixture of hydrocarbons having a larger number of carbon atoms than the n-paraffin used as the feed. Since the hydrocarbon mixture thus formed has a high octane number and boils substantially in the gasoline range, this side reaction is considered very valuable in cases where the isomerization process is aimed at the manufacture of a gasoline having a high octane number.

As was already observed hereinbefore, the catalytic dehydrogenation of paraffins to form olefins also plays an important role in the manufacture of gasoline. On the one hand, the olefins formed can be converted into alkylate gasoline by reaction with iso-paraffins ; on the other hand, under the influence of catalysts suitable for the purpose, they can be converted into an aromatic hydrocarbon mixture substantially boiling in the gasoline range. The paraffins that are subjected to the dehydrogenation treatment preferably contain at most four and in particular three or four carbon atoms per molecule. The dehydrogenation of the low-molecular-weight paraffins mentioned above may be carried out at a temperature of 400-700 °C, a pressure of 0.1-10 bar and a space velocity of 0.5-15 $g \cdot g^{-1} \cdot h^{-1}$. The dehydrogenation is preferably carried out at a temperature of 500-600 °C, a pressure of 0.5-5 bar and a space velocity of 1.5-10 $g \cdot g^{-1} \cdot h^{-1}$.

In addition to their use as catalysts and catalyst carriers in a variety of catalytic processes, the crystalline cobalt silicates according to the invention are also suitable for many kinds of other applications, for instance as adsorbers and extractants, as drying agents, as ion exchangers and as molecular sieves. An example of the latter type of application is the separation of p-xylene from mixtures of p-xylene with o-xylene and m-xylene.

The invention is now elucidated with the aid of the following example :

## Example

Four crystalline silicates (silicates 1-4) were prepared by heating, in water, in an autoclave under autogenous pressure for 24 hours at 150 °C, mixtures of NaOH, $(C_3H_7)_4NOH$, amorphous silica containing 20 ppmw Al (for the preparation of silicate 1) or amorphous silica containing 300 ppmw Al (for the preparation of silicates 2-4) and optionally $NaAlO_2$ (for the preparation of silicates 3 and 4) or Co(III) pentadione (for the preparation of silicate 1). Upon cooling of the reaction mixtures the silicates formed were filtered off, washed with water until the pH of the wash water was about 8 and dried for two hours at 120 °C. After one hour's calcination in air at 500 °C silicates 1-4 had the following properties :

a) thermally stable up to a temperature of at least 800 °C,

b) an X-ray powder diffraction pattern substantially corresponding with that given in Table B,

c) a value for m as recorded in Table C.

Table C

| Silicate No. | $SiO_2/Co_2O_3$ | $SiO_2/Al_2O_3$ |
|---|---|---|
| 1 | 76 | 36000 |
| 2 | -- | 2250 |
| 3 | -- | 290 |
| 4 | -- | 170 |

The molar composition of the aqueous mixtures from which silicates 1-4 were prepared may be rendered as follows :

$$x\ Na_2O \cdot y\ [(C_3H_7)_4N]_2O \cdot z\ Co_2O_3 \cdot p\ Al_2O_3 \cdot 25\ SiO_2 \cdot 450\ H_2O,$$

where x, y, z and p have the values listed in Table D.

Table D

| Silicate No. | x | y | z | p |
|---|---|---|---|---|
| 1 | 1 | 1.5 | 0.33 | - |
| 2 | 3 | 4.5 | - | 0.008 |
| 3 | 1 | 4.5 | - | 0.063 |
| 4 | 1 | 1.5 | - | 0.125 |

From silicates 1-4 were prepared silicates 5-8, respectively, by boiling silicates 1-4 with a 1.0 molar $NH_4NO_3$ solution, washing with water, boiling again with a 1.0 molar $NH_4NO_3$ solution and washing, drying at 120 °C and calcining at 500 °C. In seven experiments silicates 5-8 were tested as catalysts for the isomerization of n-hexane and for the dehydrogenation of isobutane.

The isomerization experiments were carried out in a 50 ml reactor containing a fixed catalyst bed of 7.5 ml volume. In four experiments n-hexane was passed over each of silicates 5-7 at different temperatures, a pressure of 5 bar, a space velocity of 1 g n-hexane per g catalyst per hour and using helium as a diluent in a He/n-$C_6$ molar ratio of 4 : 1. The results of these experiments, averaged over the first 10 hours, are recorded in Table E.

(See Table E page 7)

Table E

| Experiment No. | Silicate No. | Temperature, °C | Conversion of n-$C_6$, w% | Isomerizing selectivity, % | Cracking selectivity, % | $C_6^+$ selectivity, % |
|---|---|---|---|---|---|---|
| 1 | 5 | 500 | 28.3 | 51.6 | 28.6 | 19.8 |
| 2 | 5 | 530 | 50.0 | 24.6 | 37.0 | 38.4 |
| 3 | 6 | 500 | 29.0 | 3.4 | 95.2 | 1.4 |
| 4 | 7 | 450 | 97.0 | 1.2 | 89.3 | 9.3 |

$$\text{Isomerizing selectivity} = \frac{\text{quantity by weight of iso-hexanes formed}}{\text{quantity by weight of n-hexane converted}} \times 100$$

$$\text{Cracking selectivity} = \frac{\text{quantity by weight of hydrocarbons formed having 5 and fewer carbon atoms}}{\text{quantity by weight of n-hexane converted}} \times 100$$

$$C_6^+ \text{ selectivity} = \frac{\text{quantity by weight of hydrocarbons formel having 6 and more carbon atoms to the exclusion of iso-hexane}}{\text{quantity by weight of n-hexane converted}} \times 100$$

The dehydrogenation experiments were carried out in a 50 ml reactor containing a fixed catalyst bed of 5 ml volume. In three experiments isobutane was passed over each of the silicates 5 and 8 at a temperature of 550 °C, a pressure of 1.5 bar and at different space velocities. The results of these experiments, averaged over the first 10 hours, are recorded in Table F.

(See Table F page 9)

Table F

| Experiment No. | Silicate No. | Space velocity, $g.g^{-1}.h^{-1}$ | Conversion of iso-$C_4$ %w | Dehydrogenating selectivity, % | Cracking selectivity, % | Selectivity (%) towards $H_2/n\text{-}C_4/C_5^+$ respectivity |
|---|---|---|---|---|---|---|
| 5 | 5 | 8 | 23.5 | 92.8 | 2.1 | 3.0/ 1.7/ 0.4 |
| 6 | 5 | 2 | 50.9 | 69.3 | 10.4 | 2.8/16.3/ 1.2 |
| 7 | 8 | 2 | 64.9 | 9.3 | 66.7 | 2.0/ 4.3/17.7 |

$$\text{Dehydrogenating selectivity} = \frac{\text{quantity by weight of butenes formed}}{\text{quantity by weight of isobutane converted}} \times 100$$

$$\text{Cracking selectivity} = \frac{\text{quantity of hydrocarbons formed having 3 and fewer carbon atoms}}{\text{quantity by weight of isobutane converted}} \times 100$$

$$\text{Selectivity towards } H_2/n\text{-}C_4/C_5^+\text{, resp.} = \frac{\text{quantity by weight of, resp., } H_2\text{/n-butane/hydrocarbons having 5 and more carbon atoms}}{\text{quantity by weight of isobutane converted}} \times 100$$

Of Experiments 1-7 only Experiments 1, 2, 5 and 6 are experiments according to the invention. Experiments 3, 4 and 7 are outside the scope of the invention. They have been included in the patent application for comparison. The results recorded in Tables E and F clearly demonstrate the good catalytic properties of the crystalline cobalt silicates according to the invention in comparison with the performance of closely related crystalline aluminium silicates.

**Claims**

1. Crystalline metal silicates which have, after one hour's calcination in air at 500 °C, the following properties :

a) thermally stable up to a temperature of at least 600 °C,

b) an X-ray powder diffraction pattern in which the four lines listed in Table A are the strongest lines

Table A

| d(Å) | Relative intensity |
|---|---|
| 11.1 ± 0.2 | VS |
| 10.0 ± 0.2 | VS |
| 3.84 ± 0.07 | S |
| 3.72 ± 0.06 | S |

in which the letters used have the following meanings : VS = very strong ; S = strong,

c) in the formula which represents the composition of the silicate expressed in moles of the oxides and which, in addition to oxides of hydrogen, alkali metal and/or alkaline earth metal, silicon and trivalent cobalt, optionally contains one or more oxides of a trivalent metal A chosen from the group formed by aluminium, iron, gallium, rhodium, chromium and scandium, the $SiO_2/(Co_2O_3 + A_2O_3)$ molar ration (m) is higher than 10 and the $A_2O_3/Co_2O_3$ molar ratio is lower than 1.

2. Crystalline silicates as claimed in claim 1, in which the overall formula m has a value between 20 and 400.

3. Crystalline silicates as claimed in claim 1 or 2, in which as a trivalent metal they contain only cobalt.

4. Crystalline silicates which have been obtained by replacing at least part of the alkali metal and/or alkaline earth metal ions present in a silicate as claimed in any one of claims 1-3, by other cations.

5. Crystalline silicates as claimed in claim 4, in which the alkali metal content is less than 0.05 %w.

6. Catalysts consisting at least partly of a silicate as claimed in any one of claims 1-5, which silicate is loaded, if desired, with one or more catalytically active metal components.

7. A process for the preparation of crystalline silicates as claimed in any one of claims 1-3, in which an aqueous mixture containing the following components : one or more compounds of an alkali metal and/or alkaline earth metal (M), one or more compounds containing an organic cation (R) or from which such a cation is formed during the preparation of the silicate, one or more silicon compounds, one or more compounds containing cobalt in the trivalent form and, if desired, one or more compounds containing a trivalent metal A, in which mixture the various compounds are present in the following ratios, expressed in moles of the oxides :

$M_{2/n}O : SiO_2 = 0.01\text{-}0.35$,
$R_{2/q}O : SiO_2 = 0.01\text{-}0.4$,
$SiO_2 : (Co_2O_3 + A_2O_3) > 10$,
$A_2O_3 : Co_2O_3 < 1$ and
$H_2O_3 : SiO_2 = 5\text{-}65$

(n is the valency of M and q is the valency of R) is maintained at an elevated temperature until the silicate has formed and in that the silicate is subsequently isolated from the mother liquor.

8. A process as claimed in claim 7, in which the aqueous mixture contains M in a sodium compound and R in a tetrapropyl ammonium compound.

9. A process for carrying out catalytic conversions, which involves the use of a catalyst as claimed in claim 6.

10. A process as claimed in claim 9, in which n-paraffins having five or six carbon atoms per molecule are isomerized by contacting them with a catalyst as claimed in claim 6 at a temperature of 450-550 °C, a pressure of 1-5 bar and a space velocity of 0.5-1.5 $g \cdot g^{-1} \cdot h^{-1}$.

11. A process as claimed in claim 9, in which paraffins having three of four carbon atoms per

molecule are dehydrogenated by contacting them with a catalyst as claimed in claim 6 at 500-600 °C, a pressure of 0.5-5 bar and a space velocity of 1.5-10 $g \cdot g^{-1} \cdot h^{-1}$.

## Ansprüche

1. Kristalline Metallsilikate, die nach einstündiger Kalzinierung an Luft bei 500 °C die folgenden Eigenschaften aufweisen :

a) thermisch stabil bis zu einer Temperatur von wenigstens 600 °C,

b) ein Röntgen s trahlen-Pulverdiffraktionsmuster, in welchem die in Tabelle A angeführten vier Linien die stärksten Linien sind :

Tabelle A

| d(Å) | Relative Intensität |
|---|---|
| 11,1 ± 0,2 | VS |
| 10,0 ± 0,2 | VS |
| 3,84 ± 0,07 | S |
| 3,72 ± 0,06 | S |

worin die verwendeten Buchstaben die folgenden Bedeutungen haben : VS = sehr stark ; S = stark,

c) in der Formel, welche die Zusammensetzung des Silikats, ausgedrückt in Molen der Oxide, darstellt und welche, zusätzlich zu Oxiden von Wasserstoff, Alkalimetall und/oder Erdalkalimetall, Silizium und dreiwertigem Kobalt, gewünschtenfalls ein oder mehrere Oxide eines dreiwertigen Metalles A enthält, ausgewählt aus der aus Aluminium, Eisen, Gallium, Rhodium, Chrom und Scandium gebildeten Gruppe, beträgt das $SiO_2/(Co_2O_3 + A_2O_3)$-Mol-Verhältnis (m) mehr als 10 und das $A_2O_3/Co_2O_3$-Mol-Verhältnis ist niedriger als 1.

2. Kristalline Silikate nach Anspruch 1, in welchen in der Summenformel m einen Wert zwischen 20 und 400 besitzt.

3. Kristalline Silikate nach Anspruch 1 oder 2, in welchen als ein dreiwertiges Metall nur Kobalt zugegen ist.

4. Kristalline Silikate, die durch Ersetzen wenigstens eines Teiles der in einem Silikat, wie in einem der Ansprüche 1 bis 3 beansprucht, vorliegendem Alkalimetall- und/oder Erdalkalimetallionen durch andere Kationen erhalten worden sind.

5. Kristalline Silikate nach Anspruch 4, in welchen der Alkalimetallgehalt weniger als 0,05 Gew.-% beträgt.

6. Katalysatoren, welche wenigstens teilweise aus einem Silikat gemäß einem der Ansprüche 1 bis 5 bestehen, welches Silikat gewünschtenfalls mit einer oder mit mehreren katalytisch aktiven Metallkomponenten beladen ist.

7. Ein Verfahren zur Herstellung von kristallinen Silikaten, wie in einem der Ansprüche 1 bis 3 beansprucht, in welchem ein wässeriges Gemisch, enthaltend die folgenden Komponenten : eine oder mehrere Verbindungen eines Alkalimetalles und/oder Erdalkalimetalles (M), eine oder mehrere Verbindungen, enthaltend ein organisches Kation (R), oder aus welcher ein solches Kation während der Herstellung des Silikates gebildet wird, eine oder mehrere Silizium-Verbindungen, eine oder mehrere Verbindungen, die Kobalt in dreiwertiger Form enthalten, und, gewünschtenfalls, eine oder mehrere Verbindungen, die ein dreiwertiges Metall A enthalten, in welchem Gemisch die verschiedenen Verbingungen in den folgenden Verhältnissen, ausgedrückt in Molen der Oxide, zugegen sind :

$M_{2/n}O : SiO_2 = 0{,}01\text{-}0{,}35,$
$R_{2/q}O : SiO_2 = 0{,}01\text{-}0{,}4,$
$SiO_2 : (Co_2O_3 + A_2O_3) > 10,$
$A_2O_3 : Co_2O_3 < 1$ und
$H_2O_3 : SiO_2 = 5\text{-}65$

(n ist die Valenz von M und q ist die Valenz von R),
bei einer erhöhten Temperatur gehalten wird, bis das Silikat ausgebildet ist, und das Silikat anschließend aus der Mutterlauge abgetrennt wird.

8. Ein Verfahren nach Anspruch 7, in welchem das wässerige Gemisch M in einer Natrium-Verbindung und R in einer Tetrapropylammonium-Verbindung enthält.

9. Ein Verfahren zur Ausführung von katalytischen Umwandlungen, welches die Verwendung eines Katalysators nach Anspruch 6 umfaßt.

10. Ein Verfahren nach Anspruch 9, in welchem n-Paraffine mit 5 oder 6 Kohlenstoffatomen je Molekül durch Inberührungbringen mit einem Katalysator nach Anspruch 6 bei einer Temperatur von 450

bis 550 °C, einem Druck von 1 bis 5 bar und einer Raumgeschwindigkeit von 0,5-1,5 $g \cdot g^{-1} \cdot h^{-1}$ isomerisiert werden.

11. Ein Verfahren nach Anspruch 9, in welchem Paraffine mit drei oder vier Kohlenstoffatomen je Molekül durch Inberührungbringen mit einem Katalysator nach Anspruch 6 bei 500 bis 600 °C, einem Druck von 0,5 bis 5 bar und einer Raumgeschwindigkeit von 1,5-10 $g \cdot g^{-1} \cdot h^{-1}$ dehydriert werden.

**Revendications**

1. Des silicates cristallins de métaux qui ont, après calcination pendant une heure dans l'air à 500 °C, les propriétés suivantes :

a) ils sont thermiquement stables jusqu'à une température d'au moins 600 °C,

b) ils ont un diagramme de diffraction des rayons X par la méthode des poudres dans lequel les quatre lignes indiquées dans le Tableau A sont les lignes les plus intenses

Tableau A

| d(Å) | Intensité relative |
|---|---|
| 11,1 ± 0,2 | VS |
| 10,0 ± 0,2 | VS |
| 3,84 ± 0,07 | S |
| 3,72 ± 0,06 | S |

où les lettres utilisées ont les significations suivantes : VS = très forte ; S = forte,

c) dans la formule qui représente la composition du silicate exprimée en moles des oxydes et qui, en plus d'oxydes d'hydrogène, de métal alcalin et/ou de métal alcalino-terreux, de silicium et de cobalt trivalent, contient éventuellement un ou plusieurs oxydes d'un métal trivalent A choisi dans le groupe formé par l'aluminium, le fer, le gallium, le rhodium, le chrome et le scandium, le rapport molaire $SiO_2/(Co_2O_3 + A_2O_3)$ (m) est supérieur à 10 et le rapport molaire $A_2O_3/Co_2O_3$ est inférieur à 1.

2. Des silicates cristallins selon la revendication 1, dans lesquels dans la formule globale m a une valeur comprise entre 20 et 400.

3. Des silicates cristallins selon la revendication 1 ou 2, qui comme métal trivalent contiennent seulement du cobalt.

4. Des silicates cristallins qui ont été obtenus en remplaçant au moins une partie des ions de métal alcalin et/ou de métal alcalino-terreux présents dans un silicate selon l'une quelconque des revendications 1-3 par d'autres cations.

5. Des silicates cristallins selon la revendication 4, dans lesquels la teneur en métal alcalin est inférieure à 0,05 % en poids.

6. Des catalyseurs constitués au moins partiellement d'un silicate selon l'une quelconque des revendications 1-5, lequel silicate supporte, si on le désire, un ou plusieurs constituants métalliques catalytiquement actifs.

7. Un procédé pour la préparation de silicates cristallins tels que revendiqués dans l'une quelconque des revendications 1-3, dans lequel un mélange aqueux contenant les constituants suivants : un ou plusieurs composés d'un métal alcalin et/ou d'un métal alcalino-terreux (M), un ou plusieurs composés contenant un cation organique (R) ou à partir duquel un tel cation est formé durant la préparation du silicate, un ou plusieurs composés du silicium, un ou plusieurs composés contenant du cobalt dans la forme trivalente et, si on le désire, un ou plusieurs composés contenant un métal trivalent A, mélange dans lequel les divers composés sont présents dans les rapports suivants, exprimés en moles des oxydes :

$M_{2/n}O : SiO_2 = 0,01\text{-}0,35$,
$R_{2/q}O : SiO_2 = 0,01\text{-}0,4$,
$SiO_2 : (Co_2O_3 + A_2O_3) > 10$,
$A_2O_3 : Co_2O_3 < 1$ et
$H_2O_3 : SiO_2 = 5\text{-}65$

(n est la valence de M et q est la valence de R)
est maintenu à une température élevée jusqu'à ce que le silicate soit formé et le silicate est ensuite séparé de la liqueur-mère.

8. Un procédé selon la revendication 7, dans lequel le mélange aqueux contient M dans un composé du sodium et R dans un composé de tétrapropyl ammonium.

9. Un procédé pour effectuer des conversions catalytiques, qui comporte l'utilisation d'un catalyseur selon la revendication 6.

10. Un procédé selon la revendication 9, dans lequel des n-paraffines ayant cinq ou six atomes de

carbone par molécule sont isomérisées par mise en contact avec un catalyseur selon la revendication 6 à une température de 450-550 °C, une pression de 1-5 bars et une vitesse spatiale de 0,5-1,5 g · $g^{-1}$ · $h^{-1}$.

11. Un procédé selon la revendication 9, dans lequel des paraffines ayant trois ou quatre atomes de carbone par molécule sont déshydrogénées par mise en contact avec un catalyseur selon la revendication 6 à 500-600 °C, sous une pression de 0,5-5 bars et à une vitesse spatiale de 1,5-10 g · $g^{-1}$ · $h^{-1}$.